# EUROPEAN PATENT SPECIFICATION

(11) **EP 2 311 394 B1**
(45) Date of publication and mention of the grant of the patent: **14.06.2017**
(21) Application number: 10187430.3
(22) Date of filing: 13.10.2010
(51) Int. Cl.: A61B 17/3211, A61B 17/32, A61B 17/34, A61B 17/22, A61B 90/00

(54) **Cannulated arthroscopic knife**
Arthroskopisches Kanülenmesser
Couteau arthroscopique canulé

(30) Priority: 14.10.2009 US 251614 P
(43) Date of publication of application: 20.04.2011
(73) Proprietor: Arthrex, Inc., Naples, FL 34108-1945 (US)
(72) Inventor: Sluss, Robert K., Naples, FL 34109 (US)
(74) Representative: Shanks, Andrew

(56) References cited:
- WO-A2-2007/100581
- US-A1- 2004 181 246
- US-A1- 2009 157 110
- US-B1- 6 296 651

## Description

### FIELD OF THE INVENTION

The present invention relates to surgical devices and, more particularly, to a cannulated dilatation sleeve combined with a knife.

### BACKGROUND OF THE INVENTION

During surgery (such as hip arthroscopy, for example), portals or small incisions are made on the skin to pass small instruments into the joint. These portals or small incisions arc typically made in a "stepwise" fashion. The surgeon will insert first a spinal needle into the joint, and then a flexible wire into the joint through the spinal needle. The flexible wire serves as a place marker, but also allows for easy passage of cannulated instruments over the wire and into the joint.

An important step during hip arthroscopy is introducing a long handled scalpel (or knife) into the joint through a portal, to cut the hip capsule. The thick hip capsule normally restrains the movement of instruments and, when a small window is cut into the thick capsule, the cut allows significantly more freedom of movement of instruments in the constrained hip joint.

Once a small window has been cut, the surgeons introduce a long blade or knife through a cannula into the joint, to protect the surrounding soft tissue and neurovascular structures, or they simply put the blade through the skin portal directly into the joint and take a chance.

US2004/0181246A1 discloses a scalpel guide system for a bladed surgical instrument in which a pair of guide wire attach to a scalpel for controlled travel of the scalpel along the wire. US6296651 discloses a positioning catheter, anchor means and a cutting catheter. The cutting catheter includes a plurality of blades and is advanceable through a deployment lumen of the positioning catheter. Advancement of the cutting catheter through the deployment lumen causes a distal end of the cutting catheter to project laterally from the positioning catheter.

Accordingly, there is a need for a combined cannulated dilation sleeve with a knife (scalpel) that is integral with the cannulated sleeve. Also needed is an improved tissue penetration device that minimizes the risk of damaging adjacent organs and tissue during the cutting or puncturing step. There is also a need for a simplified surgical procedure to form a cut or puncture within tissue.

### SUMMARY OF THE INVENTION

The present invention is defined in the appended claims. The present invention comprises a cannulated dilation sleeve combined with (integrated with) a knife and having the features of claim 1. In an exemplary embodiment, the knife is configured to retract up into the body of the sleeve. The sleeve is a double lumen tube to allow the knife to retract within one of the lumens of the tube. The sleeve is mounted on a handle provided with a spring or friction slide to advance and retract the knife blade barrel. The integrated cannula/knife is configured to be placed over a guide wire.

The combined cannula/knife system of the present disclosure saves significant time during surgery, as the cannula does not require a separate step for being introduced before the knife, since the cannula and the knife are combined. The combined cannula/knife system of the present disclosure also protects soft tissue and organs adjacent the cut, as the knife is retracted up into the protective sleeve once it has been introduced into the patient.

The present disclosure also describes a method of penetrating tissue by *inter alia*: (i) providing a combined cannulated dilation sleeve integrated with a knife, in the proximity of tissue to be cut or punctured; and (ii) deploying the knife from a lumen of the sleeve to cut or puncture tissue.

In an example only, a method of portal placement and capsular resection during hip arthroscopy according to the present disclosure comprises the steps of: (i) introducing a spinal needle; (ii) introducing a flexible guide wire through the needle; (iii) removing the needle, leaving the guide wire in place; (iv) sliding a cannulated retractable arthroscopic knife over the guide wire and into the joint, with the knife blade retracted back into sleeve; (v) removing the guide wire; (vi) deploying the knife to cut the capsule; (vii) retracting the knife blade into the housing, and reintroducing the guide wire through the knife cannulation; and (viii) removing the retractable knife from the joint, leaving the guide wire in place. The described methods are not part of the claimed invention, but help to understand its advantages and application.

These and other features and advantages of the invention will be more apparent from the following detailed description that is provided in connection with the accompanying drawings and illustrated exemplary embodiments of the invention.

### BRIEF DESCRIPTION OF THE DRAWINGS

FIG. 1 illustrates a combined cannulated sleeve/knife according to an exemplary embodiment of the present disclosure.
FIG. 2 illustrates an enlarged view of the distal end of the knife assembly received in the combined cannulated sleeve/knife of FIG. 1, without the knife blade installed.
FIG. 3 illustrates an enlarged view of the distal end of the knife assembly received in the combined cannulated sleeve/knife of FIG. 1, with the knife blade installed.
FIG. 4 illustrates an enlarged view of the distal end of the combined cannulated sleeve/knife of FIG. 1.
FIG. 5 illustrates an enlarged end view (taken from the most distal end) of the combined cannulated sleeve/knife of FIG. 1.
FIG. 6 illustrates a side view of the combined cannulated sleeve/knife of FIG. 1 and placed over a guide wire.
FIG. 7 illustrates a front end view of the tubular guide or sleeve in an embodiment of the invention with separate cannulas for the knife assembly and guide wire.
FIG. 8 illustrates a side cross-sectional view of the tubular guide or sleeve of FIG. 7.
FIG. 9 illustrates a perspective view of the tubular guide or sleeve of FIG. 7 with a guide wire inserted therethrough.
FIG. 10 illustrates a perspective view of the combined cannulated sleeve/knife embodiment of FIG. 7 with a guide wire and knife assembly extending through respective cannulas and with the knife in a deployed or operative position.

### DETAILED DESCRIPTION OF THE PREFERRED EMBODIMENTS

The present disclosure provides systems and methods for penetrating tissue. The system of the present disclosure comprises a tissue penetrating assembly comprising a cannulated dilation sleeve integrated with a knife. In an exemplary embodiment, the knife is configured to retract up into the body of the sleeve. The sleeve is a double lumen tube to allow the knife to retract within one of the lumens of the tube. The sleeve is mounted on a handle provided with a spring or friction slide to advance and retract the knife blade barrel. The integrated cannula/knife is configured to be placed over a guide wire.

The combined cannula/knife system of the present disclosure saves significant time during surgery, as the cannula needs not be introduced before the knife, since the cannula and the knife are combined. The combined cannula/knife system of the present disclosure also protects the soft tissue and organs adjacent the cut, as the knife is retracted up into the protective sleeve once it has been introduced into the patient.

The present disclosure also provides a method of penetrating tissue by *enter alia*: (i) providing a combined cannulated dilation sleeve integrated with a knife in the proximity of tissue to be cut or punctured; and (ii) deploying the knife from a lumen of the sleeve to cut or puncture tissue.

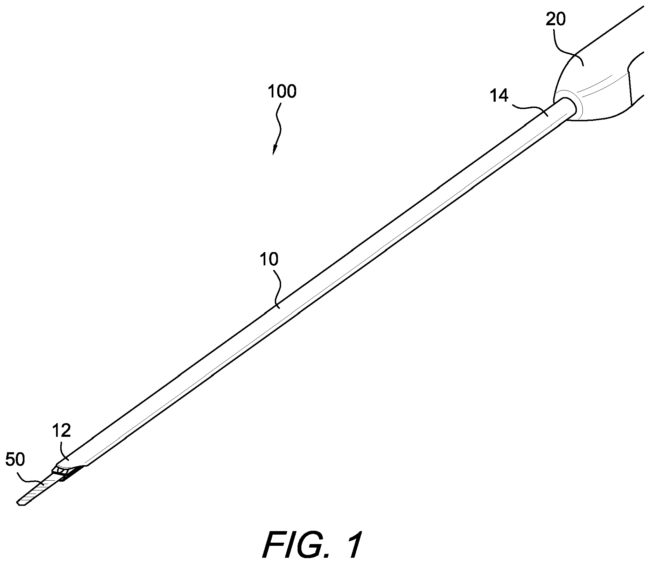

Referring now to the drawings, where like elements are designated by like reference numerals, Figures 1-10 illustrate a combined cannulated sleeve/knife 100 of the present disclosure formed of a tubular member or cannulated sleeve 10 (cannulated dilation sleeve 10) which receives a knife assembly 5 with a knife 50 (blade or scalpel 50) configured to retract within a lumen of the tubular member or sleeve 10. Instrument 100 also includes a handle 20 (Figure 1) securely attached to the tubular member or cannulated sleeve 10 and provided with an operating mechanism (such as a trigger, spring or friction slide, for example) configured to actuate (advance or retract) the knife assembly 5 or, more preferably, to advance or retract the cannulated sleeve relative to the knife assembly. In this manner, the blade or scalpel 50 may be disposed in at least two positions relative to the tubular member of sleeve 10: a retracted or non-working position (in which the knife assembly 5 with blade 50 is partially or totally retracted within the lumen of the tubular member or sleeve 10) and an extended or working position (in which the knife assembly 5 and blade 50 partially or totally extends a distance beyond the most distal end of the tubular member or sleeve 10).

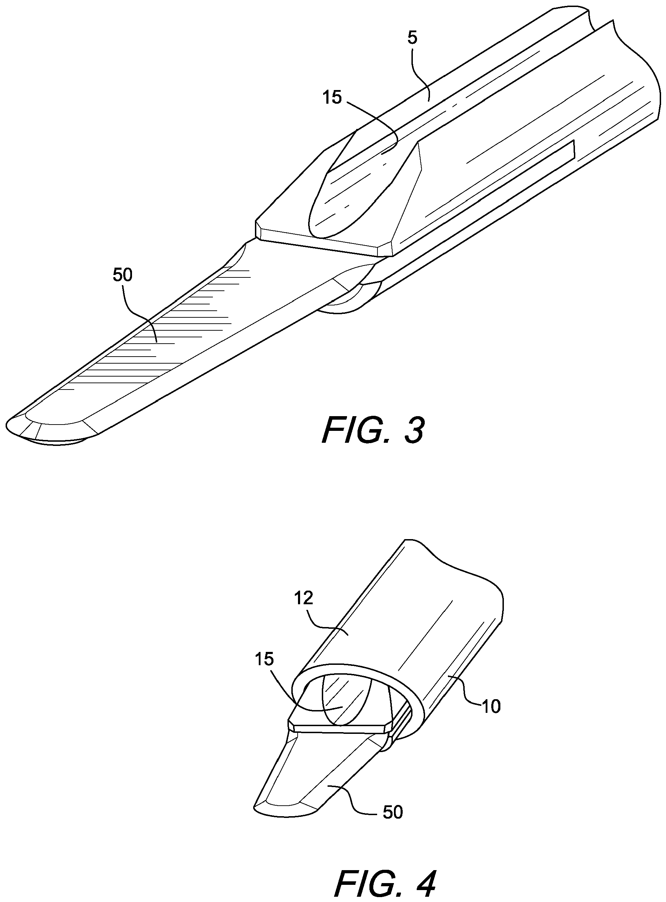

As shown in Figure 1, the tubular member or cannulated sleeve 10 has a distal end 12 and a proximal end 14. Knife or blade 50, mounted on knife assembly 5, is configured to exit a lumen at a most distal end 12of the sleeve 10 and to extend at least a distance beyond the most distal end 12of the sleeve (as shown in Figure 4, for example). The tubular member or cannulated sleeve 10 may be formed from a medically acceptable material such as stainless steel, and may have a round, cylindrical or ellipsoidal cross-section, among others. Preferably, the cannulated sleeve is tubular and has a round cross-section.

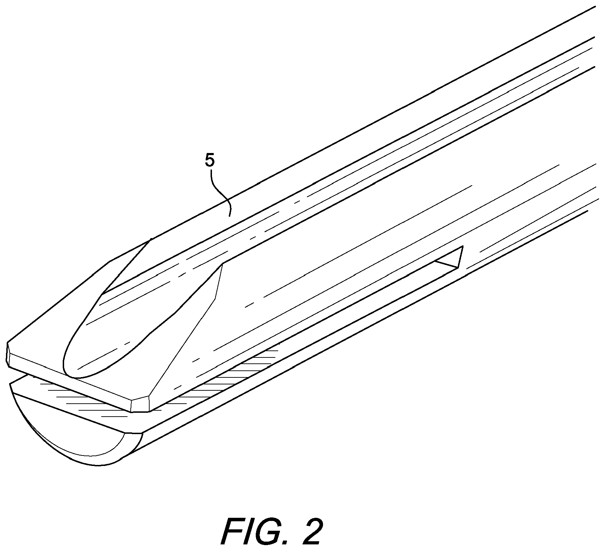

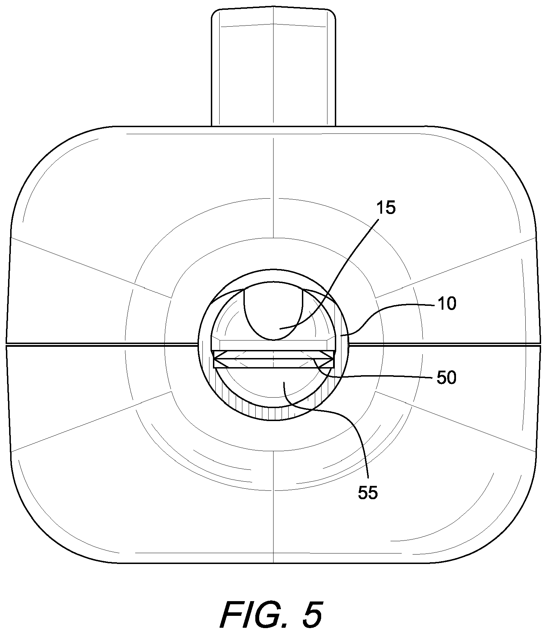

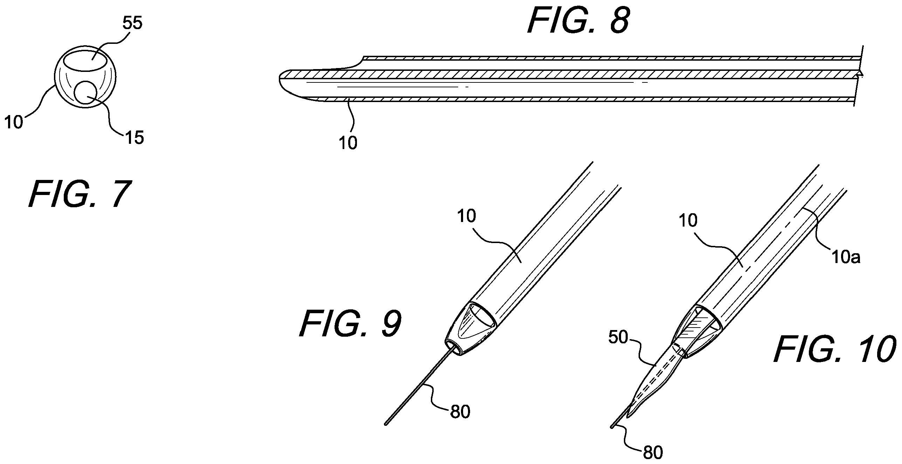

Figure 2 illustrates knife assembly 5 without a knife or blade 50 installed (pre-assembly), while Figure 3 shows knife assembly 5 fully assembled with knife 50. Figure 4 illustrate knife or blade 50 deployed from the cannulated sleeve 10 (in a working, cutting or extended position). Figure 5 illustrates a view taken from the most distal end 12 of the sleeve 10. When the knife or blade 50 is in the non-working or non-cutting (inoperative) position, the blade is at least partially (preferably totally) shielded within a lumen of the cannulated sleeve 10. When the knife or blade 50 is in the working or cutting (operative) position, the blade is projected from the lumen of the cannulated sleeve 10. In this manner, the blade is protected when the knife is not being used or is being retrieved prior to the actual cutting. The user may then choose between the retracted blade position (shown in Figure 2) and the extended, deployed position (shown in Figures 3 and 4). In the extended (working or cutting position), blade 50 is about parallel to (aligned with) a longitudinal axis 10a (shown schematically in Figure 10) of the cannulated sleeve 10.

As shown in Figures 5 and 7, sleeve 10 is configured as a multiple-lumen tube stock, for example, as a double-lumen tube 10 comprising two lumens or two cannulations, i.e., a first lumen or cannulation 55 adjacent a second lumen or cannulation 15 (which can be formed in the knife assembly 5, as shown). In an exemplary embodiment, one of the first and second cannulations 55, 15 houses a first surgical instrument (for example, a cutting blade or scalpel 50) and the other of the first and second cannulations 55, 15 allows passage of a second surgical instrument (for example, a guide wire 80) or may act as an aspiration/irrigation port (so that any fluid and/or loose tissue resulting from the cutting procedure can be aspirated through one of the lumens of the multiple-lumen sleeve 10).

In an exemplary embodiment, the first lumen or cannulation 55 is a blade lumen or blade cannulation 55 that houses knife assembly 5 with blade 50, and the second lumen or cannulation 15 is a guide wire lumen or guide wire cannulation 15 that houses guide wire 80. Knife assembly 5 with blade 50 is configured to slide along (to retract and exit) within the blade cannulation 55. The guide wire cannulation 15 slides over the guide wire 80.

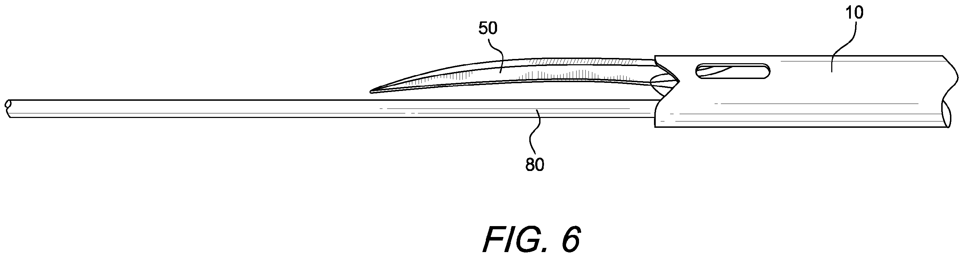

The first lumen or cannulation 55 may have a cross-section similar to, or different from, that of the second lumen or cannulation 15. In an exemplary embodiment only, and as shown in Figures 5 and 7, blade cannulation 55 may be oval or eliptical and is located adjacent the guide wire cannulation 15. Guide wire cannulation 15 may be preferably round and allows a flexible guide wire (or another surgical instrument) to be introduced therethrough. Figures 6 and 10 illustrate flexible guide wire 80 adjacent blade or knife 50 (shown in the deployed configuration), with the guide wire 80 extending about parallel to blade or knife 50 and to the longitudinal axis 12a.

In an exemplary embodiment only, the combined cannulated sleeve/knife 100 of the present disclosure is an integrated guide wire/blade device. The blade can extend through one of the lumens of the device and, in use, the blade can be selectively movable between a retracted position where the blade is in a constrained configuration within the lumen and an extended position in which a portion of the blade extends a distance beyond a most distal end of the lumen. The portion of blade that extends the distance beyond the distal end of the lumen cuts and/or penetrates tissue or structures adjacent to the tissue.

The integrated cannula/knife instrument 100 described above may be employed in various surgical medical procedures such as conventional open surgeries or in other, less invasive, techniques that use cannulas or various port access devices. The present disclosure has applications in surgical procedures where the target tissue is cut or punctured, and may be employed in cutting various body parts such as the knee, shoulder, hip, ankle, elbow, hand or foot. For example, the integrated cannula/knife instrument 100 of the present disclosure may be employed in arthroscopic surgery of a knee joint or hip structure.

A method of cutting/penetrating tissue with the cannulated arthroscopic knife of the present disclosure comprises *inter alia* the steps of:
(i) providing a combined cannulated dilation sleeve 10 integrated with a knife 50, in the proximity of tissue to be cut or punctured; and (ii) deploying the knife 50 from a lumen 55 of the sleeve 10, to cut or puncture tissue.

In an exemplary and illustrative embodiment only, a method of portal placement and capsular resection during hip arthroscopy according to the present disclosure comprises the steps of: (i) introducing a spinal needle; (ii) introducing a flexible guide wire 80 through the needle; (iii) removing the needle, leaving the guide wire 80 in place; (iv) sliding a cannulated retractable arthroscopic knife 100 over the guide wire 80 and into the joint, with the knife blade 50 retracted back into sleeve 10; (v) removing the guide wire 80; (vi) deploying the knife 50 to cut the capsule; (vii) retracting the knife blade 50 into the housing 55, and reintroducing the guide wire 80 through the knife cannulation 15; and (viii) removing the retractable knife 100 from the joint, leaving the guide wire 80 in place.

Although the present invention has been described in relation to particular embodiments thereof, many other variations and modifications and other uses will become apparent to those skilled in the art. Therefore, the present invention is to be limited not by the specific disclosure herein, but only by the appended claims.

## Claims

1. A combined cannulated dilation sleeve (100) integrated with a cutting blade (50) comprising:
a double-lumen tubular dilation sleeve (10) having a distal end (12), a proximal end (14), and a longitudinal axis (12a);
a guide wire (80);
wherein the double-lumen tubular dilation sleeve (10) comprises a first lumen (55) and a second lumen (15) adjacent to the first lumen (55), and the first lumen and the second lumen are cannulations of the dilation sleeve;
wherein one (55) of the first and second lumens (55,15) houses a cutting blade (50) having an inoperative position in which the cutting blade is retracted within the tubular dilation sleeve (10) and an operative or extended position wherein the cutting blade (50) extends a distance beyond the most distal end (12) of the double-lumen tubular dilation sleeve (10),
wherein the other of the first and second lumens (55,15) is configured to slide over the guide wire (80) and to house the guide wire (80), with the guide wire (80) extending about parallel to the cutting blade (50) and to the longitudinal axis (12a), wherein
in said operative or extended position the cutting blade (50) exits the lumen and extends about parallel to the longitudinal axis and beyond the most distal end (12) of the double-lumen tubular dilation sleeve (10),
a handle (20) is securely attached to the dilation sleeve (10) and has an operating mechanism configured to advance or retract the dilation sleeve (10) or a knife assembly (5) relative to the other of the dilation sleeve and the knife assembly, the knife assembly being received in the dilation sleeve (10) and on which said cutting blade (50) is mounted.

2. The combined cannulated dilation sleeve (100) integrated with a cutting blade (50) of claim 1, wherein the first lumen (55) has a different cross-section than the second lumen (15).

3. The combined cannulated dilation sleeve (100) integrated with a cutting blade (50) of claim 1, wherein the first lumen (55) has a circular cross-section and the second lumen (15) has an oval cross-section.

4. The combined cannulated dilation sleeve (100) integrated with a cutting blade (50) of claim 1, wherein the dilation sleeve (10) is formed of stainless steel.

## Patentansprüche

1. Kombinierte kanülierte Dilatationshülse (100), in die eine Schneidklinge (50) integriert ist, umfassend:
eine rohrförmige Doppellumendilatationshülse (10) mit einem distalen Ende (12), einem proximalen Ende (14) und einer Längsachse (12a);
einen Führungsdraht (80);
wobei die rohrförmige Doppellumendilatationshülse (10) ein erstes Lumen (55) und ein zweites Lumen (15), das dem ersten Lumen (55) benachbart ist, umfasst, und wobei das erste Lumen und das zweite Lumen Kanülierungen der Dilatationshülse sind;
wobei eines (55) des ersten und des zweiten Lumens (55, 15) eine Schneidklinge (50) aufnimmt, die eine Ruheposition, in der die Schneidklinge innerhalb der rohrförmigen Dilatationshülse (10) zurückgezogen ist, und eine Betriebs- oder ausgefahrene Position aufweist, wobei die Schneidklinge (50) um eine Distanz ausfährt, die über das distalste Ende (12) der rohrförmigen Doppellumendilatationshülse (10) hinausgeht,
wobei das andere des ersten und des zweiten Lumens (55, 15) so konfiguriert ist, dass es über den Führungsdraht (80) gleitet und den Führungsdraht (80) aufnimmt, wobei sich der Führungsdraht (80) ungefähr parallel zur Schneidklinge (50) und zur Längsachse (12a) erstreckt,
wobei:
die Schneidklinge (50) in der Betriebs- oder ausgefahrenen Position aus dem Lumen austritt und ungefähr parallel zur Längsachse und über das distalste Ende (12) der rohrförmigen Doppellumendilatationshülse (10) hinaus ausfährt,
ein Griff (20) fest an der Dilatationshülse (10) angebracht ist und einen Betriebsmechanismus aufweist, der so konfiguriert ist, dass er die Dilatationshülse (10) oder eine Messerbaugruppe (5) in Bezug auf das andere der Dilatationshülse und der Messerbaugruppe vorwärtsbewegt oder zurückzieht, wobei die Messerbaugruppe in der Dilatationshülse (10) aufgenommen ist und die Schneidklinge (50) an dieser befestigt ist.

2. Kombinierte kanülierte Dilatationshülse (100), in die eine Schneidklinge (50) integriert ist, nach Anspruch 1, wobei das erste Lumen (55) einen anderen Querschnitt als das zweite Lumen (15) aufweist.

3. Kombinierte kanülierte Dilatationshülse (100), in die eine Schneidklinge (50) integriert ist, nach Anspruch 1, wobei das
erste Lumen (55) einen kreisförmigen Querschnitt aufweist und das zweite Lumen (15) einen ovalen Querschnitt aufweist.

4. Kombinierte kanülierte Dilatationshülse (100), in die eine Schneidklinge (50) integriert ist, nach Anspruch 1, wobei die Dilatationshülse (10) aus Edelstahl gebildet ist.

## Revendications

1. Manchon de dilatation perforé combiné (100) intégré avec une lame de coupe (50) comprenant :
un manchon de dilatation tubulaire à double lumière (10) ayant une extrémité distale (12), une extrémité proximale (14), et un axe longitudinal (12a) ;
un fil de guidage (80) ;
dans lequel le manchon de dilatation tubulaire à double lumière (10) comprend une première lumière (55) et une deuxième lumière (15) adjacente à la première lumière (55), et la première lumière et la deuxième lumière sont des canules du manchon de dilatation ;
dans lequel une (55) des première et deuxième lumières (55,15) abrite une lame de coupe (50) ayant une position non fonctionnelle dans laquelle la lame de coupe est rétractée au sein du manchon tubulaire de dilatation (10) et une position opérationnelle ou étendue dans lequel la lame de coupe (50) s'étend à une distance au-delà de l'extrémité la plus distale (12) du manchon de dilatation tubulaire à double lumière (10),
dans lequel l'autre de la première et deuxième lumières (55,15) est configurée pour glisser sur le fil de guidage (80) et abriter le fil de guidage (80), avec le fil de guidage (80) s'étendant environ parallèlement à la lame de coupe (50) et à l'axe longitudinal (12a),
dans lequel dans ladite position opérationnelle ou étendue la lame de coupe (50) sort de la lumière et s'étend environ parallèlement à l'axe longitudinal et au-delà de l'extrémité la plus distale (12) du manchon de dilatation tubulaire à double lumière (10),
un manche (20) est attaché de manière sécurisée au manchon de dilatation (10) et a un mécanisme de fonctionnement configuré pour avancer ou reculer le manchon de dilatation (10) ou un ensemble de couteaux (5) par rapport à l'autre du manchon de dilatation et l'ensemble de couteaux, l'ensemble de couteaux étant reçu dans le manchon de dilatation (10) et sur lequel ladite lame de coupe (50) est montée.

2. Manchon de dilatation perforé combiné (100) intégré avec une lame de coupe (50) selon la revendication 1, dans lequel la première lumière (55) a une section transversale différente de la deuxième lumière (15).

3. Manchon de dilatation perforé combiné (100) intégré avec une lame de coupe (50) selon la revendication 1, dans lequel la première lumière (55) a une section transversale circulaire et la deuxième lumière (15) a une section transversale ovale.

4. Manchon de dilatation perforé combiné (100) intégré avec une lame de coupe (50) selon la revendication 1, dans lequel le manchon de dilatation (10) est formé d'acier inoxydable.
